# EUROPEAN PATENT APPLICATION

(11) **EP 3 677 668 A1**
(43) Date of publication of application: **08.07.2020**
(21) Application number: 18851313.9
(22) Date of filing: 30.08.2018
(51) Int. Cl.: C12N 1/21, C12N 15/63, C12N 15/70, C12Q 1/04, C12R 1/19

(54) **GENE CONSTRUCT AND BIOSENSOR FOR THE RAPID DETECTION OF AHL MOLECULES AND THE PATHOGENIC BACTERIA THAT PRODUCE SAME**

(30) Priority: 30.08.2017 CL 20172201
(71) Applicant: Universidad de Valparaiso, 2361845 Valparaìso (CL)
(72) Inventor: DINAMARCA, Alejandro, Valparaíso 2391415 (CL); ROMO, Natalia, Valparaíso 2391415 (CL); IBACACHE, Claudia, Valparaíso 2391415 (CL)
(74) Representative: Herrero & Asociados, S.L.
(86) International application number: PCT/CL2018/050077
(87) International publication number: WO 2019/041058

(57) **Abstract**

The invention relates to a gene construct for detecting the presence of microorganisms that produce chemical molecules of the Acyl-homoserine lactone (AHL) type, comprising a first expression cassette that includes a copper-inducible promoter operably linked to the gene encoding the RhIR protein and, downstream of said first expression cassette, a second expression cassette including a promoter that is induced by the AHL-RhIR complex, said promoter being operably linked to a gene encoding a reporter protein. The invention also includes a genetically modified biosensor cell to detect the presence of microorganisms comprising said gene construct, as well as the method of detecting the presence of microorganisms that produce AHL-type chemical molecules by immobilizing the biosensor in an organic matrix, generating a surface that can be used to expose a sample of a fluid such as air or a liquid medium, and thus detect these molecules mediated by the biosensor cell.

LISTADO DE SECUENCIAS

<110> UNIVERSIDAD DE VALPARAêSO

<120> CONSTRUCCIîN G\203NICA Y BIOSENSOR PARA LA RçPIDA DETECCIîN DE
MOL\203CULAS AHLs Y LAS BACTERIAS PATîGENAS QUE LAS PRODUCEN

<130> 1702-974-PCT

<150> CL 2201-2017
<151> 2017-08-03

<160> 1

<170> PatentIn version 3.5

<210> 1
<211> 1824
<212> ADN
<213> Secuencia Artificial

<220>
<223> Construcci\227n g\216nica

<400> 1
agcgcagggc gcgccggtcg gcgcgcccta ccagatctgg caggttgcct gccgttcatc 60

ctcctttagt cttccccctc atgtgtgtgc tgctctttaa tttatcagat ccaaggaggt 120

ttgacctatg gtgagcaagg gcgaggagct gttcaccggg gtggtgccca tcctggtcga 180

gctggacggc gacgtaaacg gccacaagtt cagcgtgtcc ggcgagggcg agggcgatgc 240

cacctacggc aagctgaccc tgaagttcat ctgcaccacc ggcaagctgc ccgtgccctg 300

gcccaccctc gtgaccaccc tgacctacgg cgtgcagtgc ttcagccgct accccgacca 360

catgaagcag cacgacttct tcaagtccgc catgcccgaa ggctacgtcc aggagcgcac 420

catcttcttc aaggacgacg gcaactacaa gacccgcgcc gaggtgaagt tcgagggcga 480

caccctggtg aaccgcatcg agctgaaggg catcgacttc aaggaggacg gcaacatcct 540

ggggcacaag ctggagtaca actacaacag ccacaacgtc tatatcatgg ccgacaagca 600

gaagaacggc atcaaggtga acttcaagat ccgccacaac atcgaggacg gcagcgtgca 660

gctcgccgac cactaccagc agaacacccc catcggcgac ggccccgtgc tgctgcccga 720

caaccactac ctgagcaccc agtccgccct gagcaaagac cccaacgaga agcgcgatca 780

catggtcctg ctggagttcg tgaccgccgc cgggatcact ctcggcatgg acgagctgta 840

caagtaatcg atccatagta caactatgcg cgcgcgcata atagcgcgcg cgctacttct 900

actgtaaaaa aaaaatgaca attttgtcat ttttctgtca ccggaaaatc agagcctggc 960

gagtaaagtt ggcggctggg tcttattact ctctggctct ttaatttatc agatccaagg 1020

aggtttgacc tatgaggaat gacggaggct ttttgctgtg gtgggacggt ttgcgtagcg 1080

agatgcagcc gatccacgac agccagggcg tgttcgccgt cctggaaaag gaagtgcggc 1140

gcctgggctt cgattactac gcctatggcg tgcgccacac gattcccttc acccggccga 1200

agaccgaggt ccatggcacc tatcccaagg cctggctgga gcgataccag atgcagaact 1260

acggggccgt ggatccggcg atcctcaacg gcctgcgctc ctcggaaatg gtggtctgga 1320

gcgacagcct gttcgaccag agccggatgc tctggaacga ggctcgcgat tggggcctct 1380

gtgtcggcgc gaccttgccg atccgcgcgc cgaacaattt gctcagcgtg ctttccgtgg 1440

cgcgcgacca gcagaacatc tccagcttcg agcgcgagga aatccgcctg cggctgcgtt 1500

gcatgatcga gttgctgacc cagaagctga ccgacctgga gcatccgatg ctgatgtcca 1560

acccggtctg cctgagccat cgcgaacgcg agatcctgca atggaccgcc gacggcaaga 1620

gttccgggga aatcgccatc atcctgagca tctccgagag cacggtgaac ttccaccaca 1680

agaacatcca gaagaagttc gacgcgccga acaagacgct ggctgccgcc tacgccgcgg 1740

cgctgggtct catctgatct atcattgtat aattatgcgc gcgcgcataa tagcgcgcgc 1800

gctacttcta ctgtaaaaaa aaaa 1824

## Description

### BACKGROUND OF THE INVENTION

The invention refers to a new biosensor system for warning of the presence of pathogenic microorganisms present in the air, surfaces or liquid fluids, which are the main agents of the infections acquired in hospital facilities or closed centers with massive attendance of people such as hotels, buildings with enclosed spaces, trains, airplanes, airports, schools, facilities of food processing or pharmaceutical, etc.

The infectious pathologies are on the increase due to multiple causes, including resistance to antibiotic therapies, climate change and modern lifestyles, related to transportation and the tendency to inhabit, work, entertain or produce in enclosed, air-conditioned spaces, unventilated and lacking of natural light which increases the risk of the presence and dissemination of risk pathogenic agents.

For instance, among the highest risk spaces are the buildings of public or private health systems designated for patients, companions, medical team and management personnel to stay for different periods of time. The risk is based on that they are spaces of attendance and concentration of people in different health conditions and, therefore, it is associated with the fact that they are also spaces of concentration and dissemination of risk microorganisms for human health.

Among the most adverse effects are those that occur within the hospital environments, where the acquisition of infectious diseases is increasingly frequent and, therefore, is an important public health problem since it involves a high cost of social resources both for the countries as for the families and the patients. An example of this is the increase on the number of days designated to the hospitalization as a result of the acquisition of pathogens within the health facilities, as well as the vital risk suffered by patients due to their condition and the complexity of drug and antibiotic treatments for determined types of antibiotic resistant pathogens.

An appropriate strategy to avoid the massive concentration and dissemination of pathogens causing infections of greater complexity, such as those acquired in internal spaces of buildings such as hospital centers (waiting rooms, hospitalization rooms, surgical wards and offices in general), hotels, food factories and pharmaceutical laboratories, is the appropriate monitoring and preventive control that allows to identify in the shortest possible time the levels and types of human pathogenic microorganisms, present in the circulating air (of closed and open spaces), surfaces, food and liquids that may get in contact with people.

The technologies available for detecting bacteria in the air or fluids in general are of two types: culture-dependent and culture-independent. The culture-dependent techniques are based on the use of a sample taking procedure and microbiological analysis based on enrichment and/or selective culture media for the growth and detection of microbial colonies for their subsequent identification. The culture-independent techniques are based on molecular identification by amplification and sequencing of ribosomal DNA or by the detection of molecules of biological origin such as the presence of ATP.

The most common technologies currently available for detecting, quantifying or identifying bacteria are:
1. Sampling of surfaces for microbiological analysis using swabs;
2. Sampling of surfaces for the detection of ATP by luminescence using the luciferase enzyme.
3. Sampling of air using anemometers for the collection and capture bacteria for the subsequent quantification of Colony Forming Unit (CFU) per volume of sampled air.
4. Identification by microbiological analysis based on technologies such as PCR and flow cytometry.

However, these techniques are not entirely adequate mainly due to:
1. The time to obtain the results of the culture-dependent techniques is not fast enough. Current technologies take an average of 2 to 5 days to detect a specific pathogen, this due to processes that involve sampling, growth and subsequent identification. This causes that when identifying the presence of a specific pathogen, this has multiplied several times and thus increased its infective capacity, affecting a greater number of people. The low specificity and high complexity of culture-independent technologies: in which rapid sampling techniques as flow cytometry are used, which only allow the general detection of total viable cells of bacteria, being therefore of low specificity. The same occurs with the ATP detection technique from surfaces, which although it is a quick analysis, turns out to be unspecific. The complexity of specific identification techniques such as amplification and sequencing of ribosomal DNA by Polymerase Chain Reaction (PCR), which involves a greater complexity of sampling and processing and, therefore, a longer time for analysis and delivery of results.

Besides of the above-mentioned, in the prior art some information has been disclosed on detection of microorganisms by the use of bacterial biosensors, such as the one the present invention. These biosensors capable of detecting specific molecules of microbial origin are genetically modified bacteria with constructs containing reporter genes under the control of an inducible promoter that responds to an effector protein activated by specific molecules, as described in documents CN103215214, Steindler, L., et al. (2007) and Lindsay, A., et al., (2005) which are summarized below.

Patent application CN103215214 refers to a gene construct with two reporter systems that allow to detect specific molecules that participate, as signals, in the bacterial cellular communication known as *quorum sensing* (QS), which in turn allows to identify the producing bacteria. The gene construct of the publication comprises an *Escherichia coli* strain, modified with a double gene regulation circuit based on inducible promoters by molecules of N-Acyl-homoserine lactones (AHLs) type and associated with the reporter genes Cherry Red Fluorescent Protein (RFP) and Green Fluorescent Protein (GFP). The inducible promoters of this regulation circuit of document CN103215214 do not consider copper or any other metal as a regulation factor, consequently they do not anticipate the biosensor of the present invention.

The detection of molecules of AHLs type is relevant due to is a biological indicator that establishes the presence bacteria that are risky for people's health. This is explained due to the QS system activated by AHLs is associated with the formation of biofilms and the genes expression associated with the virulence in a great diversity of pathogenic bacteria for humans, such as: *Pseudomonas aeruginosa, Staphylococcus aureus, Clostridium difficile or Vibrio cholerae* among others. Likewise, the state of the art discloses that the type of AHL molecule is specifically related with to the producing microorganism.

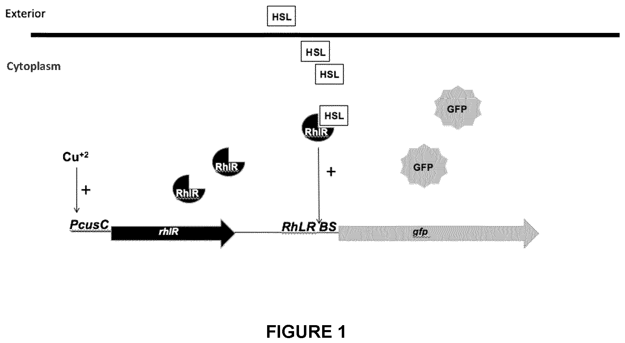

On the other hand, the publication of Steindler, L., et al., (FEMS Microbiol. Lett., 266:1 - 9; 2007), reviews the state of the art regarding the detection of AHLs, where different systems based on gene constructs are described that comprise a promoter regulated by exogenous AHLs, capable of activating different reporter genes associated with clearly detectable phenotypes such as; bioluminescence, β-galactosidase activity, fluorescence (Green Fluorescence Protein GFP) or color (by violacein pigment) (Fig.1). These phenotypes may be detected by luminometric, colorimetric, fluorometric or chromatographic type techniques, allowing to indirectly quantify the presence of AHLs molecules. Steindler, L., *et al*., do not describe any system based on a double gene construct of an inducible first and a second promoter, and where one of them is activated by copper and directs the expression of the *rhlr* gene, therefore, it does not disclose nor does it suggest any gene construct as the one of the present invention.

The publication of Lindsay A., et al., (J. Bacteriology, 187: 5054-58; 2005), presents a study to determine the effect of the sdiA gene on the expression of transcription factors that respond to AHLs, using four gene constructs, where one of them, expresses the *rhlr* gene under the control of its homologous promoter. The difference with the invention proposed here is that Lindsay, A., *et al* does not describe either any system based on a double gene construct of an inducible first and second promoter, where one of them is activated by an inorganic element as copper and that directs the expression of the gene that encodes for RhIR. Finally, Lindsay, A., *et al*., also does not consider to include the expression of a reporter gene such as gfp or Lux genes that are incorporated in our invention to obtain a quick fluorescence or luminescence signal, which is already according to the current technology, easily detectable and quantifiable by fluorometer or luminometric equipment known to date.

Among those systems described in the documents recently summarized and that could be considered the closest to the proposed invention, it is known to obtain strains of bacteria (mainly *E*. *coli*), transformed into biosensors capable of detecting and informing the presence of AHLs, but in the systems of gene expression of those previous works, in none of them it is incorporated - nor is it suggested to incorporate -, a promoter induced by copper or any other metal.

The low efficiency in the early and specific detection of the current systems allows the spread of pathogenic bacteria that may even be resistant to preferred antibiotic treatments, increasing the incidence of complex infectious pathologies with a result of increased mortality rates in infected people.

According to the above, the development of new strategies that are more fast and specific is required for the identification of pathogenic microorganisms present in the air, surfaces and liquid fluids in general, in order to have greater control over them and thus, improve the protection systems of people, particularly, of those who must go and stay a long time in hospital or high human transit facilities such as airports, schools, hotels, universities, institutional buildings, shopping malls or mass transportation means as airplanes or trains, and places for animal breeding for human consumption, food production or pharmaceutical products.

The present invention solves this problem by designing and constructing of a bacterial biosensor of high specificity, useful for detecting molecules of AHLs type produced when the communication system between bacteria known as *quorum sensing* (QS) is activated, activated by high cellular densities and on which behaviors such as the formation of biofilms or the virulence of bacterial pathogens depends.

### SUMMARY OF THE INVENTION

In the present invention, the detection of the molecules is enhanced by using copper to induce the basal expression of the gene that encodes the effector protein RhIR, capable of interacting with AHLs activating a second gene expression system, which it may be associated with a reporter gene (which lacks of its promoter) to send fluorescent, luminescent or colorimetric type signals, depending on the gene chosen. The biosensor of the present invention may be immobilized in organic polymer matrices containing an appropriate culture media and that include agar-agar or agarose in sufficient quantities that allow to quantify signals, for example fluorescence, when it gets in contact with AHL-producing bacteria, such as *Pseudomonas aeruginosa.* The immobilized or in suspension cells of the biosensor may be used for detecting AHL produced by bacteria present in the air or in different matrices.

Particularly, the present invention relates to a gene construct designed and synthesized to detect Acyl-homoserine lactone (AHL) in fluid media, which comprises two expression cassettes in tandem, where a first expression cassette includes a copper-inducible promoter, operably linked to the gene encoding RhIR protein, and downstream of said first expression cassette, a second expression cassette including a promoter that is induced by AHL-RhIR complex, said promoter being operably linked to a gene that encodes a reporter protein.

In a preferred embodiment of the invention, the copper-inducible promoter of the gene construct is the *pcusC* promoter.

In another preferred embodiment of the invention, the reporter protein whose expression is induced by the AHL-RhIR complex is selected from the group consisting of proteins that emit fluorescence, luminescence and color, and more preferably is the green fluorescent protein (GFP).

The gene construct of the present invention has the nucleotide sequence identified as SEQID No.1.

The invention also includes a plasmid to detect the presence of Acyl-homoserine lactones (AHL) in fluids media, which comprises the gene construct of the invention.

Another object of the invention is a biosensor cell genetically modified to detect the presence of Acyl-homoserine lactones (AHL) in fluid media comprising a gene construct or a plasmid that contains a first expression cassette including a copper-inducible promoter operably linked to the gene that encodes RhIR protein, and downstream of said first expression cassette, a second expression cassette including an AHL-RhIR complex-inducible promoter, said promoter being operably linked to a gene that encodes a reporter protein.

In a preferred embodiment of this invention object, said biosensor cell is a bacterium, more preferably is the *Escherichia coli* bacterium and in an even further preferred manner, is the MG1655 pUCPAO1RHL strain of *Escherichia coli,* deposited in the Microbial Genetic Resources Bank with access number RGM 2382.

A last object of the invention is a method to detect the presence of Acyl-homoserine lactones (AHL) in fluid media, said method comprising the stages of:
- providing a suspension of biosensor cells that comprise a gene construct or a plasmid containing a first expression cassette that includes a copper-inducible promoter operably linked to the gene encoding the RhIR protein, and, downstream of said first expression cassette, a second expression cassette including an AHL-RhIR-inducible promoter, said promoter being operably linked to a gene encoding a reporter protein;
- immobilizing said biosensor cells in an organic matrix;
- exposing said immobilized biosensor cells to the fluid in which the presence of Acyl-homoserine lactones (AHL) is to be detected;
- culturing the biosensor cells exposed to the presence of Acyl-homoserine lactones in a culture medium containing copper; and
- determining the presence of Acyl-homoserine lactones (AHL) in the fluid by the emission of a fluorescent, luminescent, or colorimetric signal, from the culture of the biosensor cells corresponding to the expression of the reporter protein.

In a preferred embodiment of the invention method, the suspension of biosensor cells to which the fluid is exposed, where the presence of Acyl-homoserine lactones (AHL) is to be detected comprises between 1 and 5 x 10⁷ cells/mL. In another embodiment of said method, biosensor cells exposed to the presence of Acyl-homoserine lactones are cultured in a medium containing copper between 100 and 1000 µM, for 6 to 12 hours at 37° C.

### DESCRIPTION OF THE FIGURES

**Figure 1****.** Shows the design of the gene construct of the invention, activated by copper for the detection of homoserine lactones by means of fluorescence emission. In the presence of copper, the *pcusC* promoter is activated by activating the transcription of the *rhlr* gene encoding for an effector regulatory protein, which by being in the presence of molecules of N-Acyl-homoserine lactones (AHLs) type, activates the promoter that directs the expression of a reporter gene that lacks of its promoter region. In this case, the reporter gene is the *gfp* gene that encodes for the green fluorescent protein (GFP).

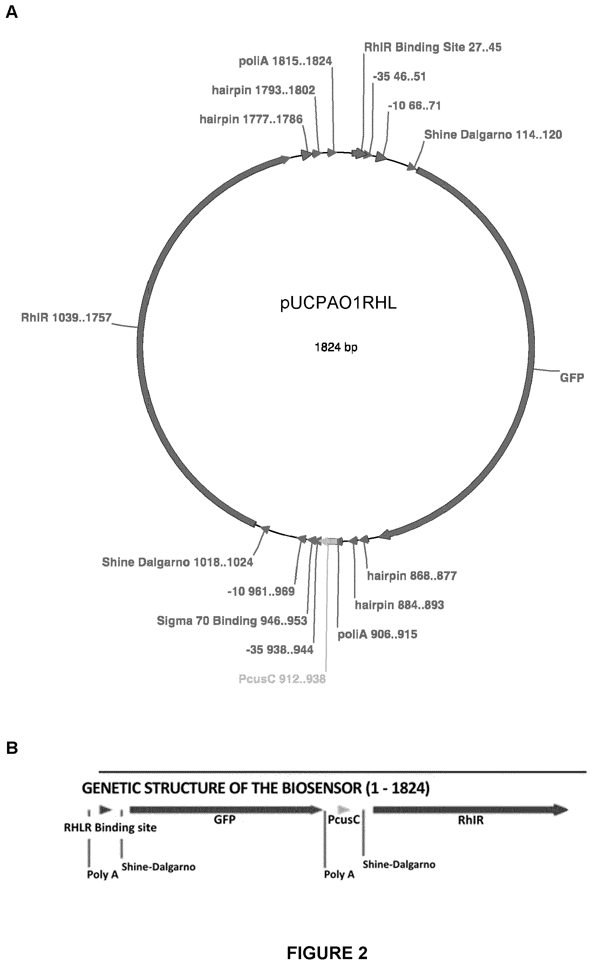

**Figure 2****.** In A, it is observed the map of the plasmid used to clone the designed and synthesized gene construct.
   In B, it is shown the map of the gene construct of the invention for detecting N-Acyl-homoserine lactones (AHLs) of the microorganism of clinical importance *Pseudomonas aeruginosa.* Said gene construct is cloned to give rise to the pUCPAO1RHL plasmid.

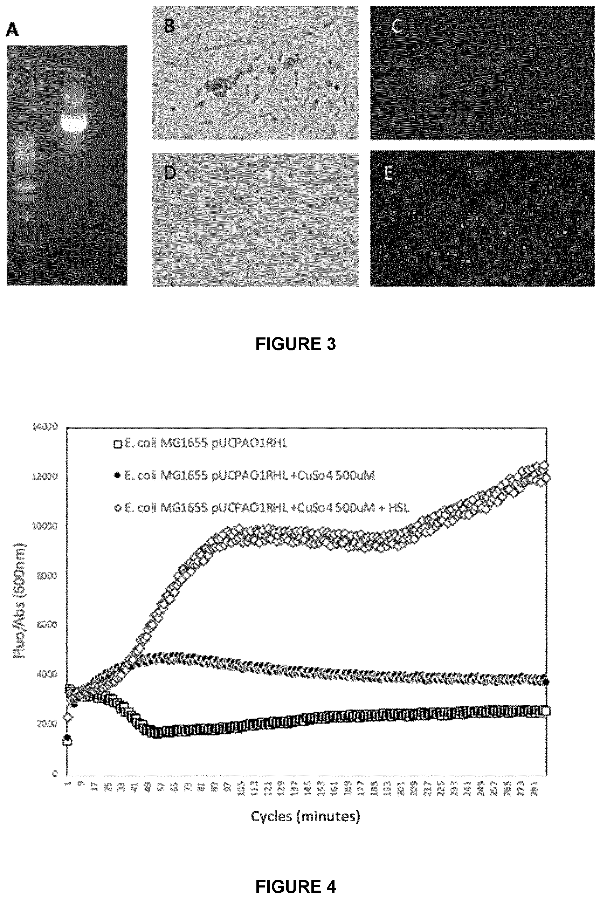

**Figure 3** **(A-E).** Plasmid pUCPAO1RHL obtained from the biosensor strain and fluorescence emission test in response to AHL. In A, a photograph of the agarose gel with molecular weight marker (left lane) and the vector pUCPAO1RHL isolated from *E*. *coli* DH5a cells (right lane) are shown. In B is shown a phase-contrast microphotograph of *E*. *coli* cells containing the plasmid pUCPAO1RHL and that were cultured in Luria-Bertani medium (LB) without copper or the presence of Acyl-homoserine lactones (AHL). In C, it is shown the same microscopic field in epifluorescence phase of *E*. *coli* cells containing the plasmid pUCPAO1RHL and that were cultured in Luria-Bertani medium without copper or the presence of N-Acyl-homoserine lactone (AHL). In D, it is observed phase-contrast microphotograph of *E*. *coli* cells containing the plasmid pUCPAO1RHL that were cultured in Luria-Bertani medium with copper and the presence of homoserine lactone obtained from a culture of *Pseudomonas aeruginosa.* In E, it is observed the microphotograph of the same field of D in epifluorescence microscopy phase of *E*. *coli* cells containing the plasmid pUCPAO1RHL and that are cultured in Luria-Bertani medium with copper and the presence of homoserine lactone, emitting fluorescence.
**Figure 4****.** The fluorescence emission response of E. coli pUCPAO1RHL strain in different conditions of chemical stimuli every 15 minutes of measurement is shown, expressed as cycles. Black circles show fluorescence (excitation 480 nm, emission 515 nm) relative to turbidity of cells suspension (Abs600nm) not exposed to N-Acyl-homoserine lactones (AHLs) of *Pseudomonas aeruginosa* and in presence of copper (CuSO4). Not-filled rhombuses show fluorescence (excitation 480 nm, emission 525 nm) relative to turbidity of cells suspension (Abs600nm) exposed to homoserine lactones (AHL) of *Pseudomonas aeruginosa* and copper (CuSO4). Not-filled squares show fluorescence (excitation 480 nm, emission 515 nm) relative to turbidity of cells suspension (Abs600nm) not exposed to N-Acyl-homoserine lactones (AHLs) of *Pseudomonas aeruginosa* or copper (CuSO4).

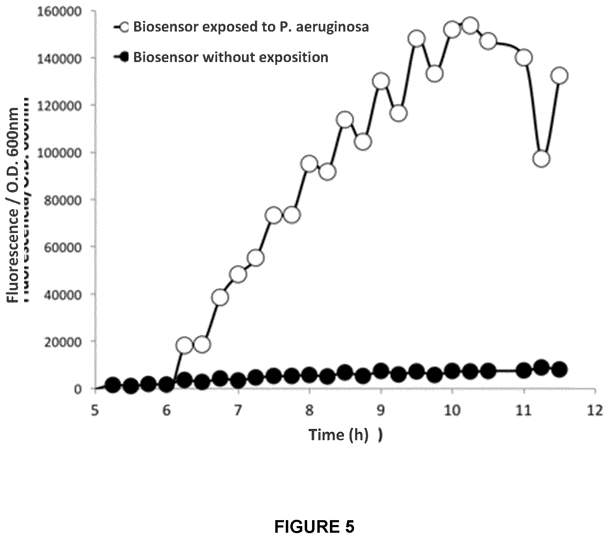

**Figure 5****.** Bioassay to assess biosensor response to direct exposition to *Pseudomonas aeruginosa.* Figure shows fluorescence emissions of the biosensor for the GFP, obtained when biosensor was exposed in liquid media to the presence of *P. aeruginosa* and copper. It is possible to discern an increase in fluorescence derived from GFP when biosensor was exposed to the presence of *P. aeruginosa* and copper (white circles).

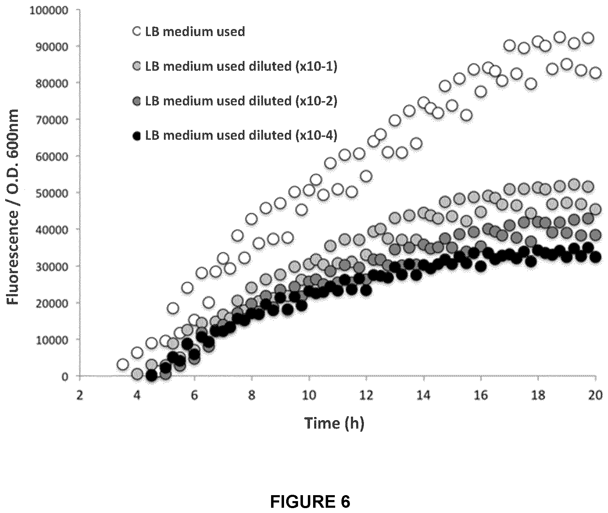

**Figure 6****.** *E*. *coli* pUCPAO1RHL biosensor response to molecules generated by *P*. *aeruginosa.*

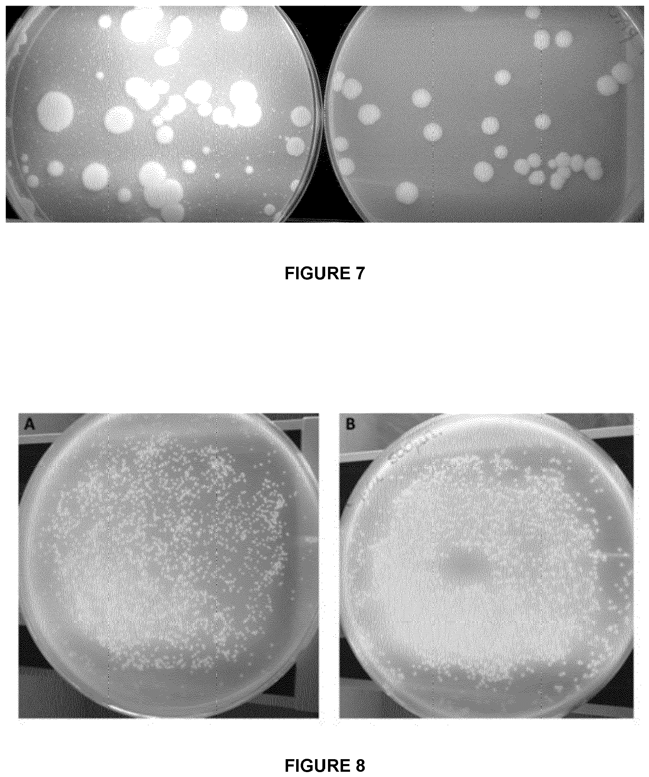

**Figure 7****.** Fluorescence generated by biosensor strain *E*. *coli* MG1655 pUCPAO1RHL as a response to its exposition to molecules produced by *P*. *aeruginosa.* The picture shows two Petri dishes of cultures, where colonies of biosensor strain grown in presence (left) and absence (right) of a LB culture media used by *P. aeruginosa* obtained from stationary phase of growth and filtrate with a pore size of 0.22 mm free of *P. aeruginosa* cells are appreciated.
**Figure 8****.** Result of detection assay of *P. aeruginosa* in air samples using *E*. *coli* pUCPAO1RHL biosensor. **A**; photograph showing colonies of *P. aeruginosa* captured from the air, in the culture media modified using sampler with anemometer. The culture media contains the biosensor in absence of copper. **B**; It corresponds to a Petri dish containing the biosensor with copper, and colonies corresponding to *P. aeruginosa* captured from air using air sampler equipped with an anemometer.

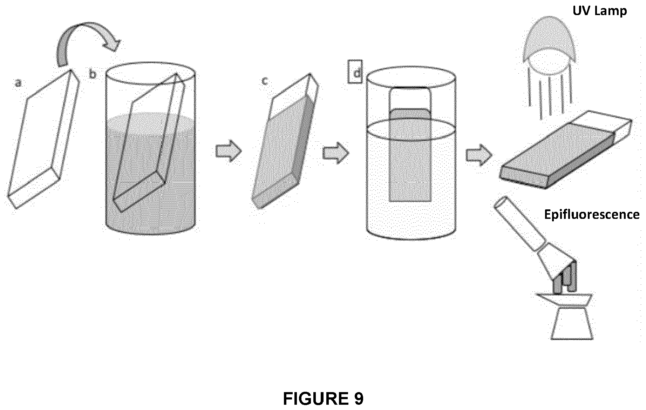

**Figure 9****.** Immobilization system of the biosensor in organic matrices for fluorescence emission assays. In (a-b), it is shown a slide-type surface, immersed in a liquid state matrix (due to temperature) containing the biosensor, copper, glucose 30 mM and the organic polymer. In this case, the polymer corresponds to agarose to 0.8 % (p/vol). The biosensor is found in a quantity of 5 x 10⁷ cells/mL. Once withdrawn, the matrix in the slide surface is solidified by cooling at room temperature, and the biosensor is immobilized on it (c). In this presentation, the biosensor was used to assess the presence of *P. aeruginosa* through AHL-type molecule detection. In order to achieve this, the slide containing the absorbed biosensor was immersed in the filtrated supernatant, free of cells of a Luria Bertani (LB) culture media, previously used for the growth of *P. aeruginosa* (d). Slides were immersed for 10 hours and they were later assessed for fluorescence emission through epifluorescence microscopy or using an UV lamp (d).

### DETAILED DESCRIPTION OF THE INVENTION

The invention proposes a biosensor which allows quick detection of pathogenic microorganism through N-Acyl-homoserine lactones (AHLs) molecules detection produced when the communication system between bacteria known as quorum sensing (QS) is activated by high cell densities, of which behaviors like biofilm formation or virulence of bacterial pathogens are dependable.

The biosensor of the invention is a bacterium containing pUCPAO1RHL plasmid within a gene construct designed and synthetized for the AHL molecules detection, which is shown in Figure 2.

Said plasmid comprises a first expression cassette including a promoter inducible by copper operatively attached to the gene that codifies RhIR protein and, downstream of said first expression cassette, a second expression cassette including a promoter induced by AHL-RhIR complex, being said promoter operatively attached to a gene that codifies a reporter protein.

In a preferred implementation of the invention, the promoter inducible by a mineral is a promoter inducible by copper, and particularly it is the *pcusC* promoter.

Thus, AHLs molecules detection is optimized by using copper to induce base expression of the gene that it codifies for the RhIR effector protein, able to interact with AHLs by activating a second system of genie expression or expression cassette that grants a detectable phenotype, by association of a reporter gene (devoid of its promoter) in order to give fluorescent, luminescent or colorimetric type signals, depending of the chosen gene (see, for example, diagram of Figure 1). In Figure 1 diagram, the activation of this reporter gene transcription generates the accumulation of green fluorescent protein, which allows detecting fluorescence. Other options of reporter genes consider Lux genes and pyoverdine synthesis. Gene construct was synthetized *de novo* and inserted in a conventional cloning vector (pUC57) to electro transforming *E*. *coli* cells.

Once expressed the reporter gene product of the presence of AHLs attached to the transcriptional activator RhIR, fluorescence, luminescence, or color, as the case may be, they can be measured and quantified using equipment as fluorescence, luminescence or color type detectors.

Biosensor of the present invention can be immobilized in organic polymeric matrices, such as agar or agarose, which contain inorganic salts and a carbon source, in amounts enough to allow quantifying fluorescence signals when entering into contact with AHLs producing-bacteria, such as *Pseudomonas aeruginosa.* Biosensor cells immobilized or in solution can be used for AHL detection from bacteria present in air or different types of samples.

Once the biosensor immobilized with the matrices to assess is contacted, the biosensor is conveniently incubated by 6 to 12 hours at 37°C and the presence of the reporter, fluorescence, color or other is subsequently determined, which can be directly correlated to the presence of pathogenic bacterium in the assessed sample.

In the biosensor regulation circuit of the present invention, RhIR effector protein responding to bacteria AHL presence, for example *P. aeruginosa* must be present in amounts enough to interact with this molecule and activate the circuit that allows the reporter expression, for example GFP, where largest concentrations of RhIR allows detecting low levels of AHL (see Figure 4). For this reason, in order to provide larger sensitivity and more specificity and control to RhIR expression, its coding sequence was brought under control of the regulating region of *cusC* gene, specifically a region containing promoter *pcusC* that in *E*. *coli* is activated in copper presence. This way, the regulation circuit of the biosensor from the invention here proposed, considers as a chemical induction condition the presence of copper to allow the expression of *rhlr* gene, activating GFP expression in presence of AHL. The state of prior art, which was already analyzed in backgrounds of the invention, even though discloses several alternatives for the pathogenic microorganism detection, is not close to the invention, which is based in a gene construct that allows the operative attaching of two different expression cassettes: the first one (for the RhIR expression) that captures communication molecules between microorganism to detect and the second one (for the GFP expression), that uses those captured communication molecules to give an immediate fluorescent signal, easy to quantify and that is proportional to the amount of microorganisms present in the media. The invention contributes a biosensor system, characterized by a high sensitivity and a fast delivery of results, allowing detecting these AHLs molecules, and therefore, producer organisms generally present in air and surfaces of hospital and public areas, giving a fast detection of pathogenic microorganisms presence.

In an implementation, the biosensor of the invention can be used in liquid fluids containing this type of molecules produced by determined types of bacteria. Fluids can be drinking water, serums used in medicine area, beverages and food in general.

In a more detailed way, the biosensor of the invention corresponds to *Escherichia coli* bacteria, which contains a pUC57 plasmid with a genetic insertion which was designed and synthetized *de novo*, which has regulatory gens and sequences that allows the cell responding to specific chemical signals when there is copper in the culture media, generating a detectable and quantifiable phenotype, for example, by fluorescence, as Figure 2 shows.

The construct designed and developed has a minimal structure, herein called expression cassette, which consists of: a DNA sequence that codifies for the RhIR transcriptional regulator; a DNA sequence of *pcusC* promotor; a DNA sequence that codifies a reporter gene, for example, green fluorescent protein (GFP) and two Shine-Dalgarno sequences. The RhIR transcriptional regulator is an effector protein that, when attaching to N-Acyl-homoserine lactones type molecules, activates transcription of specific genes. In the present invention, the DNA sequence that codifies for RhIR was brought under a regulator region which is activated by *pcusC,* which in *E*. *coli* is activated in presence of copper. In the present invention, DNA sequence that codifies for the reporter gene was brought under a regulator region which is activated by regulator RhIR attached to homoserine lactones.

In order to have, in a controlled manner, the presence of RhIR regulator protein, the DNA sequence that codifies for this protein was designed under the control of a regulator region that responds to the presence of copper, where the presence of this metal is necessary to activate and reinforce the *rhlr* gene transcription. This way, when the biosensor is in copper and N-Acyl-homoserine lactones type presence, the production of the GFP is exponentially activated, and consequently, a phenotype, that in this case is fluorescence in green color quantifiable by current systems broadly known in the state of the current technique, is emitted.

Considering that homoserine lactones are signaling molecules of the bacteria communication system known as *quorum sensing,* and that this system activates the virulence bacterial pathogens when they reach certain cellular densities, the present invention proposes this design as a method of specific detection of pathogens through a reporter signal, for example fluorescence, derived from the recognizing of specific signaling molecules of *quorum sensing.*

The main advantage this system presents is that it allows the record of pathogens presence in air samples or liquid solutions in less time than current technologies, important factor related to infectious diseases propagation within closed spaces.

Other advantage this system presents is that the RhIR regulator of *P. aeruginosa* can recognize AHLs of acyl chain through the C₄-HSL (N-butyryl-L-homoserine lactone) specific type ((doi: 10.1128/JB.183.19.5529-5534.2001; doi OI:10.1111/j.1574-6976.2001.tb00583.x; doi: 10.1038/nrm907; doi: 10.1111/j.1574-6968.2006.00501.x). This is relevant as it grants specificity regarding the detected AHL type, therefore, of the producer microorganism, considering that in accordance with the amount of carbon atoms, they can be short, medium and long, which is correlated with the type of producer bacteria.

A biosensor cell of the invention detailed throughout this memory and particularly in examples 3 and 4, has been recorded in the Coleccion Chilena de Recursos Genéticos Microbianos (Chilean Collection of Microbial Genetic Resources) under Access code RGM 2381.

Biosensor cell of the invention can be immobilized in agar or other adequate organic polymers, such as Petri dishes or coated slides. After being contacted with the sampled to obtain, the immobilized biosensor can be incubated for a period from 6 to 12 hours, after which the expression of the reporter gene is assessed. For example, if GFP is used, the fluorescence emitted after incubation can be seen or measured. In Figure 9, a diagram of an immobilization system of the biosensor in organic matrices for fluorescence emission assays is shown.

Hereafter, preferred ways of implementation of the proposed invention are shown, and even though they aim to illustrate the invention, they shall not be considered to limit their reach, which shall be determined by the accompanying claims.

### EXAMPLES

### 1. DNA sequences included in the gene construct of the invention

The selection of regulator sequences containing promoter sites of response to copper and RhIR regulator protein (able to respond to the cytoplasmic presence of chemical signals involved in *quorum sensing*) was performed from data obtained from database platforms such as Prodoric (http://www.prodoric.de), (Münch, R., Hiller, K., Barg, H., Heldt, D., Linz, S., Wingender, E. Jahn,D. (2003) PRODORIC: prokaryotic database of gene regulation. Nucleic Acids Res. 31, 266-269), Ecocyc (https://ecocyc.org) (Keseler et al. 2017, "EcoCyc: reflecting new knowledge about Escherichia coli K-12", Nucleic Acids Research 45:D543-50), BioCyc (https://biocvc.org), BPROM (http://www.softberry.com/berry.phtml?topic =ann2 ann3&no menu=on), NCBI, and Pseudomonas (pseudomonas.com).

### 2. Design and collection of the gene construct and the biosensor cell of the invention.

As previously mentioned, the gene construct of the invention was designed using genie regulator sequences answering to chemical signals and codifying of regulator proteins involved in *quorum sensing.* Additionally, constructs contain a reporter, which in this case corresponds to the sequence of the gene codifying for the *Green Fluorescence Protein* (GFP). The architecture and organization of the gene construct, when introduced in *Escherichia coli,* allow generating a biosensor cell able to respond to copper and molecules inducing the response of *quorum sensing* in bacteria, expressing a measurable and quantifiable phenotype, which in this case is detected by fluorescence emission (Figures 1, 3, and 4), through epifluorescence microscopy, a spectrofluorometer detector or a UV lamp, without excluding the possibility to incorporate other reporter gene with a quantifiable phenotype as luminescence.

Microphotographies from Figure 3 were obtained using an epifluorescence microscopy Leica (model DM4000B LED with epifluorescence). The source of AHL was the supernatant filtrated from a culture of stationary phase of *Pseudomonas aeruginosa* (1 liter), which was processed for the collection and purifying of these molecules through organic extraction (doi: 10.1128/JB. 188.2.773-783.2006). From the extract obtained, its mass was measured and then it was resuspended in a final volume of 1 mL. Bioassays were performed by exposition of 180 mL of culture exposed at 10 mL of the extract obtained from the *Pseudomonas aeruginosa* culture.

The detection system of the gene construct of the invention consists of the *pcusC* promotor sequence that in *Escherichia coli* is activated by the *cusRS* regulator system in presence of copper (Identification of a Copper-Responsive Two-Component System on the Chromosome of Escherichia coli K-12 George P. Munson, Deborah L. Lam, F. Wayne Outten, and Thomas V. O'Halloran (2000), J. Bacteriol. 182:20 5864-5871; doi:10.1128/JB.182.20.5864-5871.2000). Under this promoter, the DNA sequence codifying for the RhIR transcriptional activator cytoplasmic protein was associated (Figure 2), which when expressing, is attached to homoserine lactones type molecules and activates the associated promotor RhIR *binding site* (Medina G, Juárez K, Valderrama B, Soberón-Chávez G. Mechanism of Pseudomonas aeruginosa RhIR Transcriptional Regulation of the rhIAB Promoter. Journal of Bacteriology 2003;185(20):5976-5983. doi:10.1128/JB.185.20.5976-5983.2003). The sequence of this promoter region (RhIR *binding site*) was included in the construct to activate the transcription of the gene codifying for GFP (Figures 1 and 2).

The minimum operative unit or expression cassette contains the different sequences used in the design of the biosensor gene construct, inserted in an expression vector, which also comprises nucleotide sequences that allow the transcription and translation of the plasmid, such as:
1.- TATA box: Natural site located within the promotor area of each gene, generally between position -35 and -10, which directs the RNA polymerase to start the gene transcription.
2.- Union sequence of the sigma factor: Natural site located near TATA box, which allows the attaching of a transcriptional factor, which works jointly with the RNA polymerase to start the transcription process. A factor sigma to activate the synthesis of the first part of the plasmid during the growth phase of the invention biosensor cell was used.
3.- Shine Dalgarno: standard nucleotide sequence for *Escherichia coli* (AGGAGG) inserted prior to start codon (ATG) of a gene, that promotes the recognizing and ribosome affinity to the site near the start of the translation.

The designed and synthesized sequence map contains the respective sequences coding for GFP and RhIR along with the respective promoters. The design of the detection system included the sequence of the *pcusC* promoter which in *Escherichia coli* responds to the *cusRS* regulatory system in the presence of copper (Identification of a Copper-Responsive Two-Component System on the Chromosome of Escherichia coli K-12 George P. Munson, Deborah L. Lam, F. Wayne Outten, and Thomas V. O'Halloran J. Bacteriol. October 2000 182:20 5864-5871; doi:10.1128/JB. 182.20.5864-5871.2000). The DNA sequence encoding for the transcriptional activator cytoplasmic protein RlhR was associated with this promoter, which, when expressed, responds to the presence of homoserine lactone-type molecules and activates the associated promoter *RhIR binding site* (Medina G, Juárez K, Valderrama B, Soberón-Chávez G. Mechanism of Pseudomonas aeruginosa RhIR Transcriptional Regulation of the rhIAB Promoter. Journal of Bacteriology. 2003; 85(20): 5976-5983. doi:10.1128/JB.185.20.5976- 5983.2003). The sequence of this promoter region (*RhIR binding site*) was placed in the construct to activate the transcription of the gene encoding for the Green Fluorescence Protein (GFP).

The construct of the invention was cloned into the Sacl/Apal site of plasmid pUC57 with ampicillin resistance obtaining the vector pUCPAO1RHL (Figures 2 and 3). The reference plasmid pUC57, with Ampicillin resistance, was digested with the restriction enzymes *Apal* and Sacl, which cut at the *Multi cloning site (MCS)*, allowing the insertion of the biosensor gene construct of the invention disclosed herein. It should be noted that the working enzymes do not cut any section of the designed biosensor gene construct.

This plasmid was used to transform the *Escherichia coli* MG1655 strain by electroporation. The resulting biosensor *Escherichia coli* MG1655 / pUCPAO1RHL was selected in Petri dishes containing Luria Bertani agar with ampicillin (100 µg/mL).

### 3. Evaluation of the biosensor in response to homoserine lactones

The biosensing strain named *E*. *coli* pUCPAO1RHL that contains the gene construct of the invention, was used to evaluate its response to the presence of AHLs and the presence of copper. For the evaluation, AHLs were obtained from the bacterium *Pseudomonas aeruginosa* from a 12-hour bacterial culture in late exponential phase. For this, the supernatant was separated from the bacterial cells by centrifugation at 8,000 rpm for 15 minutes. An organic extraction with dichloromethane (DCM) from the supernatant was performed in a ratio of 70:30 (Supernatant: DCM). For this, 200 ml of supernatant and 85 ml of DCM were incorporated into a 250 ml volume decantation flask. It was stirred vigorously, then left to settle at room temperature for 1 hour. After the phases were completely separated, the organic phase was recovered, and the solvent was evaporated by means of a rotary evaporator. The extract obtained was massed and resuspended in 100 µL of DCM for subsequent analysis.

The biosensor strain (180 µL) was grown in the presence of 10 µL of AHL extract (obtained from the stationary phase of growth of *P. aeruginosa*) and in the presence of 500 µM of copper (such as CuSO₄) in Luria Bertani liquid medium at 37°C stirred in 96-well plates on a temperature controlled orbital shaker spectrofluorometer equipment (Tecan USA). During growth, turbidity was recorded at an absorbance of 600 nm and fluorescence at 420 nm every 15 minutes, equivalent to one cycle of fluorescence emission measurement.
F igure 4 shows the sensitivity and response to Cu⁺² and homoserine lactones of the biosensor *E*. *coli* MG1655 pUCPAO1RHL. The source of AHL was the supernatant filtered from a stationary phase culture of *Pseudomonas aeruginosa* (1 liter) that was processed to obtain and purify these molecules by organic extraction (doi: 10.1128/JB.188.2.773-783.2006). The obtained extract was massed and then resuspended in a final volume of 1 mL. Bioassays were performed by exposing 180 µL of a biosensor culture (Abs₆₀₀ₙₘ = 0.05) to 10 µL of the extract obtained in 96-well microtiter plates. The plates were incubated in a Tecan spectrofluorometer kit (Infinite® 200 Pro model, equipped with temperature control) at 37°C for 48 hours with absorbance and fluorescence measurements in each cycle.

The results of the fluorescence emission of the biosensor when exposed to AHL from *P. aeruginosa* and copper, summarized in Figures 3 and 4, clearly demonstrate that the biosensor cell of the invention disclosed herein allows rapid and specific detection of pathogenic bacteria that secrete *quorum sensing* molecules of the homoserine lactone-type.

Figure 4 shows clearly that it is possible to appreciate the difference in the type of relative fluorescence curves (Abs₄₂₀ₙₘ) (fluorescence/optical density Abs600nm) emitted by *E. coli* pUCPAO1RHL cells exposed or not to the presence of AHL and copper. When the cells are not exposed to AHL or copper, there is no fluorescence emission, since the gene construct contained in the plasmid pUCPAO1RHL is not activated and therefore the *gfp* gene (without its native or original promoter) is not expressed. This situation changes when the cells are exposed to AHL obtained from *P. aeruginosa* and copper simultaneously, making it possible to observe a relative fluorescence (Abs₄₂₀ₙₘ) emission curve (fluorescence/optical density Abs₆₀₀ₙₘ) showing that from cycle 17 (4.25 hours of exposure), an exponential emission phase is triggered until cycle 81 (20.25 hours of exposure) with values between 4000 and 10000. This phase was only observed in cells exposed to AHL from *P. aeruginosa* and copper. On the other hand, copper by itself did not show an obvious inducing effect of fluorescence, which is related to the fact that this chemical stimulus only allows the transcription of the RhIR regulator to be activated, which only in the presence of AHL can activate the transcription of gfp. In the absence of copper and the presence of AHL, the same low level of activation of gfp transcription is observed, demonstrating that copper exponentially activates the transcription of the RhIR regulator, and with it the ability to detect AHL from circulating *P. aeruginosa,* achieving a higher level of sensitivity, which favors the early detection of pathogenic microorganisms. It is important to note that in the aforementioned assays, a strain of *E*. *coli* that does not contain AHL-activated genes from *P. aeruginosa* was used as the host for the plasmid pUCPAO1RHL.

### 4. Evaluation of the biosensor in response to direct exposure to P. aeruginosa.

A bioassay was performed to assess the biosensor response to direct exposure to *Pseudomonas aeruginosa.* The assay was performed in a mixed culture using 96-well plates on a spectrofluorometer equipment. The plates were incubated in an Infinite® 200 Pro model spectrofluorometer equipment equipped with temperature control (Tecan) at 37°C for 48 hours with absorbance and fluorescence measurements in each cycle (GFP measured by 480nm excitation, 515nm emission, Abs₆₀₀ₙₘ turbidity). For this, a 16-hour culture of the biosensor strain was used to inoculate Luria Bertani medium at a final biosensor concentration of 10⁵ cells/ml. *P. aeruginosa* was incorporated into the culture in a final concentration of 10⁶ cells/ml. Each well of the plate was inoculated with 190 µL of biosensor + 10 µL of *P. aeruginosa* to be incubated at 37°C for 24 hrs in a Tecan spectrofluorometer (Infinite 200 Pro model). GFP expression was recorded by measuring fluorescence intensity at an excitation and emission wavelength of 480nm and 515nm respectively. Cell growth was determined by measuring absorbance at 600 nm.

The results are shown in Figure 5, which indicate that after 6 hours it is possible to clearly detect the *P. aeruginosa* strain in the culture by expression of GFP.

Secondly, a test was carried out to expose the biosensor to biomolecules produced by *P. aeruginosa* during its growth, using the supernatant from a culture of this microorganism (Figure 6). For this, a 16-hour lateexponential / early stationary phase culture of *P. aeruginosa* grown at 37°C was centrifuged at 3320 *g* at 5°C in an Eppendorf centrifuge. The obtained supernatant was filtered using 0.22 µm filters. The biosensor was directly exposed to the obtained filtered supernatant culture medium, naming it LB medium used, and in dilutions thereof of ×10-1, ×10-2, and ×10-4.

Under these conditions, the biosensor was grown for 20 hours to measure the fluorescence emission in a Tecan spectrofluorometer equipment (Infinite® 200 Pro model) equipped with temperature control at 37°C. GFP fluorescence was measured by excitation at 480nm and 515nm emission, and turbidity at an absorbance of Abs₆₀₀ₙₘ. The results are shown in Figure 6. It can be noted that even the diluted supernatants have a fluorescent response after 8 hours of incubation with the biosensor.

The results could even be seen with the naked eye, for example, in Figure 7 a culture of the strain is shown in the presence (left) and absence (right) of a LB culture medium used by *P. aeruginosa* and filtered with a pore size 0.22 µm free of *P*. *aeruginosa* cells. The used culture medium obtained after 24 hours of incubation at 37°C and where the stationary growth phase of *P. aeruginosa* was reached. Once the Petri dishes were inoculated with a biosensor inoculum, they were incubated at 37°C for 24 hours. As it can be seen, the biosensor of the invention fluoresces in the presence of the *P. aeruginosa* culture supernatant.

Figures 6 and 7 demonstrate that the biosensor is sensitive to the biomolecules produced by this microorganism, among which are AHLs.

### 5. Evaluation of the biosensor to determine the presence of P. aeruginosa from air samples

A bioassay was performed to assess whether the biosensor can be used to determine the presence of *P. aeruginosa* from air samples. In order to carry out this test, the biosensor encapsulated in an agar agar matrix is used to form a modified culture medium of the Nutritive Agar type, which also contains copper, such as copper sulfate. The encapsulated biosensor and copper were placed in a Petri dish, which was used for the sampling of microorganisms existing in the air.

For the test, it was used an air sampling equipment of MAS-100 NT brand equipped with a digital anemometer that allows to regulate the air volumes and sampling time. The sampling equipment with the Petri dish containing the medium: Luria Bertani Copper agar inoculated with the biosensor, were used to sample inside an ESCO brand Biosafety Cabinet equipment (AC14E1 model), in which a 20 liters capacity sterile bag was available. Aerosols of *P. aeruginosa* were generated inside the bag by means of a spray-type sprayer, then proceeding to sample. Once the Petri dish containing the culture medium modified with the biosensor and copper was exposed, it was incubated for at least 8 hours in an incubator at 37°C. At the end of the incubation, the plates with colonies were exposed to a portable ultraviolet light lamp equipment to detect fluorescence in the dark. As a control, a copper-free plate was used in the culture medium. Both plates were incubated for 10 hours at 37°C and finally exposed to ultraviolet (U.V.) light from a portable lamp. The results are shown in Figure 8. It can be seen that in the medium comprising copper (B) there is a much greater luminescence than in the medium without copper (A). However, the biosensor allowed to detect the presence of *P. aeruginosa* in the air in both plates.

## Claims

**1.** A gene construct for detecting Acyl-homoserine lactones (AHL) in fluid media, **CHARACTERIZED in that** it comprises a first expression cassette that includes a copper-inducible promoter operably linked to the gene encoding the RhIR protein and, downstream of said first expression cassette, a second expression cassette including a promoter that is induced by the AHL-RhIR complex, said promoter being operably linked to a gene encoding a reporter protein.

**2.** The gene construct according to claim 1 **CHARACTERIZED in that** said copper-inducible promoter is the *pcusC* promoter.

**3.** The gene construct according to claim 1, **CHARACTERIZED in that** the reporter protein is selected from the group consisting of proteins that emit fluorescence, luminescence, and color.

**4.** The gene construct according to claim 3 **CHARACTERIZED in that** the reporter protein is the green fluorescent protein (GFP).

**5.** The gene construct according to claim 1, **CHARACTERIZED in that** said gene construct has the nucleotide sequence identified as SEQID No. 1.

**6.** A plasmid for detecting the presence of Acyl-homoserine lactones (AHL) in fluid media, **CHARACTERIZED in that** it comprises a gene construct that includes a first expression cassette that includes a copper-inducible promoter operably linked to the gene encoding the RhIR protein and, downstream of said first expression cassette, a second expression cassette including a promoter that is induced by the AHL-RhIR complex, said promoter being operably linked to a gene encoding a reporter protein.

**7.** A biosensing cell genetically modified to detect the presence of N-Acyl-homoserine lactones (AHL) in fluid media, **CHARACTERIZED in that** it comprises a gene construct or a plasmid that contains a first expression cassette that includes a copper-inducible promoter operably linked to the gene encoding the RhIR protein and, downstream of said first expression cassette, a second expression cassette including a promoter that is induced by the AHL-RhIR complex, said promoter being operably linked to a gene encoding a reporter protein.

**8.** The biosensor cell according to claim 7, **CHARACTERIZED in that** said cell is a bacterium.

**9.** The biosensor cell according to claim 8, **CHARACTERIZED in that** said bacterium is *Escherichia coli.*

**10.** The biosensor cell according to claim 9, **CHARACTERIZED in that** said bacterium is the *Escherichia coli* MG1655 pUCPAO1RHL strain, deposited in the Microbial Genetic Resources Bank with access number RGM 2382.

**12.** A method to detect the presence of Acyl-homoserine lactones (AHL) in fluid media, **CHARACTERIZED in that** it comprises:
- providing a suspension of biosensor cells comprising a gene construct or a plasmid containing a first expression cassette including a copper-inducible promoter operably linked to the gene encoding the RhIR protein and, downstream of said first expression cassette, a second expression cassette including a promoter that is induced by the AHL-RhIR complex, said promoter being operably linked to a gene encoding a reporter protein;
- immobilizing said biosensor cells in an organic matrix;
- exposing said immobilized biosensor cells to the fluid in which the presence of Acyl-homoserine lactones (AHL) is to be detected;
- culturing biosensor cells exposed to the presence of Acyl-homoserine lactones in a culture medium containing copper;
- determining the presence of Acyl-homoserine lactones (AHL) in the fluid by the emission of a fluorescent, luminescent, or colorimetric signal from the culture of the biosensor cells, corresponding to the expression of the reporter protein.

**13.** The method according to claim 12, **CHARACTERIZED in that** the suspension of biosensor cells comprises between 1 and 5 x 10⁷ cells/mL.

**14.** The method according to claim 12, **CHARACTERIZED in that** the biosensor cells exposed to the presence of Acyl-homoserine lactones are cultivated in the medium containing copper between 100 to 1000 µM, for 6 to 12 hours at 37°C.
